# EUROPEAN PATENT APPLICATION

(11) **EP 1 911 388 A1**
(43) Date of publication of application: **16.04.2008**
(21) Application number: 06756897.2
(22) Date of filing: 01.06.2006
(51) Int. Cl.: A61B 1/00, G02B 23/24

(54) **ENDOSCOPE AND ENDOSCOPE INSERTION SECTION**

(30) Priority: 01.08.2005 JP 2005223192
(71) Applicant: OLYMPUS MEDICAL SYSTEMS CORP., Shibuya-ku Tokyo 151-0072 (JP)
(72) Inventor: YAMAYA, Koji, c/o OLYMPUS MEDICAL SYSTEMS CORP., Tokyo 152-0072 (JP)
(74) Representative: von Hellfeld, Axel
(86) International application number: PCT/JP2006/311020
(87) International publication number: WO 2007/015334

(57) **Abstract**

An endoscope includes: an elongate insertion portion including a bendable bending portion; an elongate flexible body as a contained matter disposed in the insertion portion, which is flexed according to a bending action of the bending portion; a protecting helical body in a helical shape formed by closely winding a wire having a predetermined elasticity, for sheathing and protecting at least a part of the flexible body that is flexed; and a regulating portion for regulating a flexion of the protecting helical body, formed by securing adjacent wires of the protecting helical body to one another.

## Description

### Technical Field

The present invention relates to an endoscope and an endoscope insertion portion provided with protecting means to protect a contained matter.

### Background Art

Endoscopes including an elongate insertion portion are widely used in inspection or the like in medical and industrial fields.

In Japanese Unexamined Patent Publication No. 2002-306404 as a first example of prior art, an endoscope is disclosed which includes an insertion portion wherein a contained matter is sheathed by a helical tube. Note that the endoscope in this Japanese Unexamined Patent Publication No. 2002-306404 is provided with a helical tube whose pitch is changed depending on the buckling force applied to the contained matter.

Further, in Japanese Unexamined Patent Publication No. 2000-214337 as a second example of prior art, an endoscope is disclosed which has an insertion portion wherein a contained matter is sheathed by a helical tube and the helical tube is filled with a soft adhesive.

In the endoscope of the first exemplary prior art, a part especially desired to be protected has a close winding. As a result, at a loose winding part, when the helical tube is bent, a large step between parts with and without a wire (non-regulating part and regulating wire part) is formed in an axial direction, which leads to a possibility that the wire at the loose winding part is caught by an adjacent member such as a bending piece. In that case, winding pitch of the helical tube is disturbed, resulting in a possible failure to obtain a predetermined protection effect and in decreased life of the contained matter.

Meanwhile, in the endoscope of the second exemplary prior art, by filling a flexible filler among wires, the part where the above-mentioned step is formed is filled with the filler, which conceivably provides an effect to prevent the catching. Further, by filling a filler into a part of a protecting coil, the flexibility of the protective coil conceivably can be changed. In that case, however, because the loose winding part not filled with the filler is exposed, the function to prevent the catching due to the step is not obtained.

Furthermore, it is also possible to combine the first and second exemplary prior arts so as to fill a filler into the loose winding part disclosed in the first exemplary prior art. In that case, it takes double labors of having to make a protecting coil including the close and loose windings that coexist, and additionally, to fill the filler into the loose winding part.

Moreover, because the position of the contained matter desired of particular protection differs for each type of endoscope depending on layout of the contained matter, structure of the bending portion, bending angle, and so on, it is costly to make the protecting coil including the close and loose windings that coexist, for each type of endoscope. In addition, although providing a close winding part allows adjusting flexibility in the axial direction, there is also a drawback that the flexibility can not be changed in line with circumferential direction.

In other words, neither of the techniques of the exemplary prior arts can inexpensively provide an endoscope having the both functions to locally protect the contained matter and to prevent catching with an adjacent member, and easily set the part desired of protection depending on the endoscope type.

The present invention has been made in view of the above-mentioned points, and an object of the present invention is to provide an endoscope and an endoscope insertion portion including a contained-matter protection function capable of protecting a contained matter, which, while maintaining the contained-matter protecting function, further locally restrict flexion, and of easily changing and setting the part desired of protection inexpensively and depending on the endoscope type.

### Disclosure of Invention

### Means for Solving the Problem

An endoscope of the present invention includes: an elongate insertion portion including a bendable bending portion; an elongate flexible body as a contained matter disposed in the insertion portion, which is flexed according to a bending action of the bending portion; a protecting helical body in a helical shape formed by closely winding a wire having a predetermined elasticity, for sheathing and protecting at least a part of the flexible body that is flexed; and a regulating portion for regulating a flexion of the protecting helical body, formed by securing adjacent wires of the protecting helical body to one another.

According to the configuration, a regulating portion to regulate flexion of the protecting helical body can be easily formed by securing adjacent wires of the protecting helical body to one another. Furthermore, there can be realized a contained-matter protection function capable of protecting a contained matter, which can, while maintaining the contained-matter protecting function, further locally restrict flexion, and can easily change and set the part desired of protection inexpensively and depending on the type of endoscope.

Moreover, the endoscope insertion portion of the present invention includes: an elongate insertion portion including a distal end portion provided with an illumination window and an observation window, a bending portion that is bendable provided at a rear end of the distal end portion, and a flexible tube portion which is elongate and has flexibility, provided at a rear end of the bending portion in a longitudinal direction of the bending portion; an elongate flexible body as a contained matter disposed in the insertion portion, which is flexed according to a bending action of the bending portion; a protecting helical body in a helical shape formed by closely winding a wire having a predetermined elasticity, for sheathing and protecting at least a part of the flexible body that is flexed; and a regulating portion for regulating a flexion of the protecting helical body, formed by securing adjacent wires of the protecting helical body to one another.

### Brief Description of the Drawings

FIG 1 is a perspective view showing an appearance of an endoscope of a first embodiment of the present invention.
FIG 2 is a vertical sectional view showing an internal configuration of a distal end side of an insertion portion.
FIG 3 is a side view showing a structure to protect a light guide fiber bundle by a protecting coil member.
FIG 4 is an illustrative view showing that position of an adhesive fixing to one another wires of the protecting coil member shown in FIG 3 is near the center in a longitudinal direction of a bending portion.
FIG 5 is a view showing the bending portion as maximally bent is fixed with the adhesive to a position where the radius of curvature of the bending portion is minimized.
FIG 6 is a view showing an exemplary fixing position different from the fixing position with the adhesive shown in FIG 3.
FIG 7 is a view showing an exemplary fixing position different from the fixing position with the adhesive shown in FIG 3.
FIG 8 is a view showing a structure where the wires of the protecting coil member are fixed to one another over the entire circumference in the circumferential direction.
FIG 9 is a view showing a structure where the wires of the protecting coil member are fixed to one another at a part in the circumferential direction.
FIG 10 is a view showing a structure where the wires of the protecting coil member are fixed to one another at a part in the circumferential direction.
FIG 11 is a vertical sectional view showing a structure near a rear end of the protecting coil member.
FIG 12 is a vertical sectional view showing the structure near the rear end of the protecting coil member in the flexible tube portion.
FIG 13 is an illustrative view of a working when a rigid portion is provided to the rear end compared to when the rigid portion is not provided.
FIG 14 is a view showing a schematic configuration where a first bending portion and a second bending portion are provided on a distal end side of an insertion portion in a second embodiment of the present invention.
FIG 15 is an illustrative view showing a schematic shape of bending pieces when the first bending portion in bent in a downward direction by 90 degrees, and the second bending portion in an upward direction by 90 degrees.
FIG 16 is a view showing a configuration where an endoscope insertion assisting tool is attached to a distal end side of an insertion portion in an endoscope apparatus of a third embodiment of the present invention.
FIG 17 is a view showing a state where a balloon of the endoscope insertion assisting tool is moved forward from the state of FIG 16.
FIG 18 is a view showing a state where, after the balloon is inflated in the state of FIG 17, an operation is performed to draw a shaft so as to haul an intestinal tract.
FIG 19 is a view showing a state where, in the state of FIG 18, the bending portion is bent, the balloon is contracted, and then the balloon is moved forward.
FIG 20 is a view showing a state where the balloon is inflated in the state of FIG 19.
FIG 21 is a view showing a state where an operation is performed to draw the shaft in the state of FIG 20 so as to haul the intestinal tract.
FIG 22 is a view showing a configuration of a modification example of FIG 16.

### Best Mode for Carrying Out the Invention

Referring to the drawings, embodiments of the present invention are described below.

### (First Embodiment)

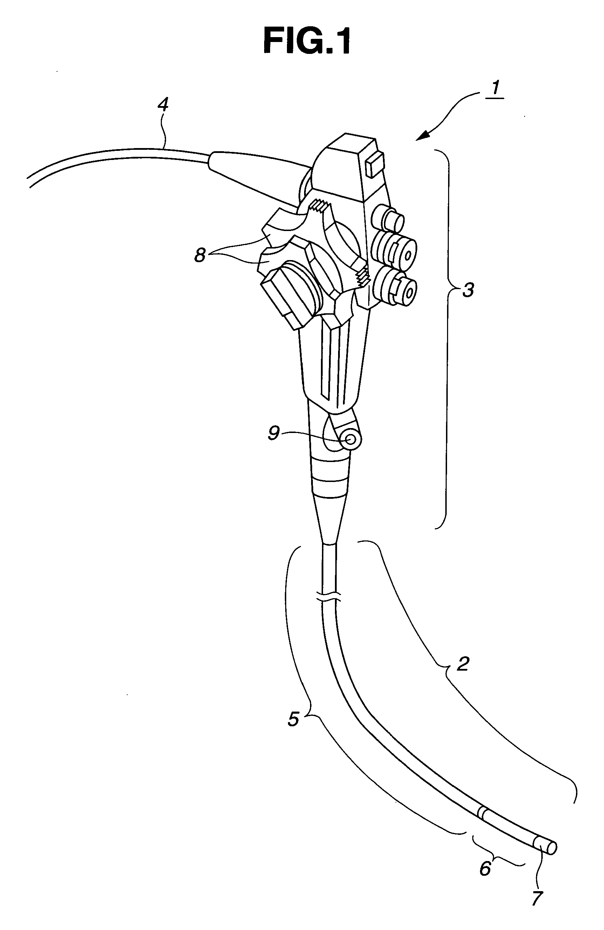

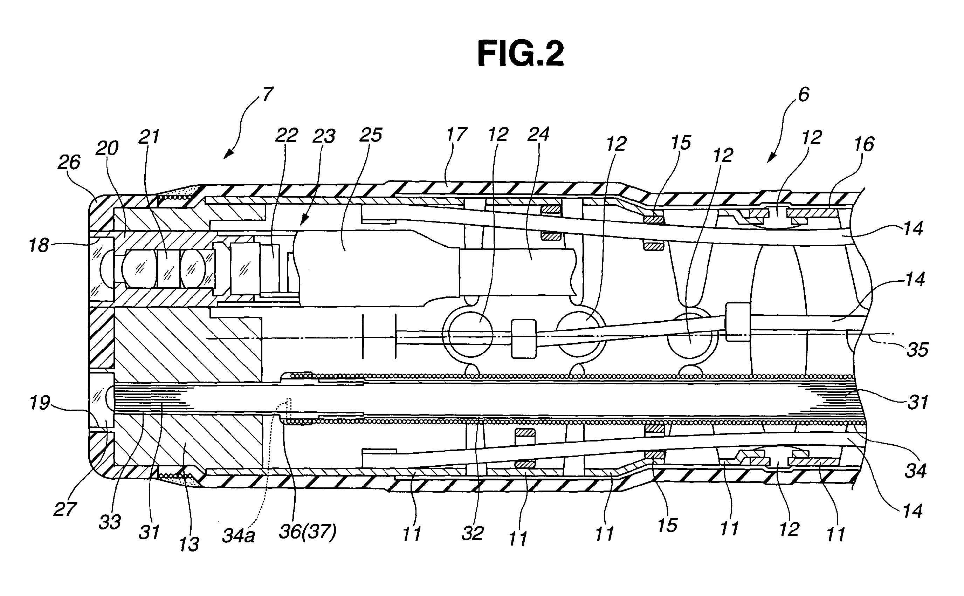

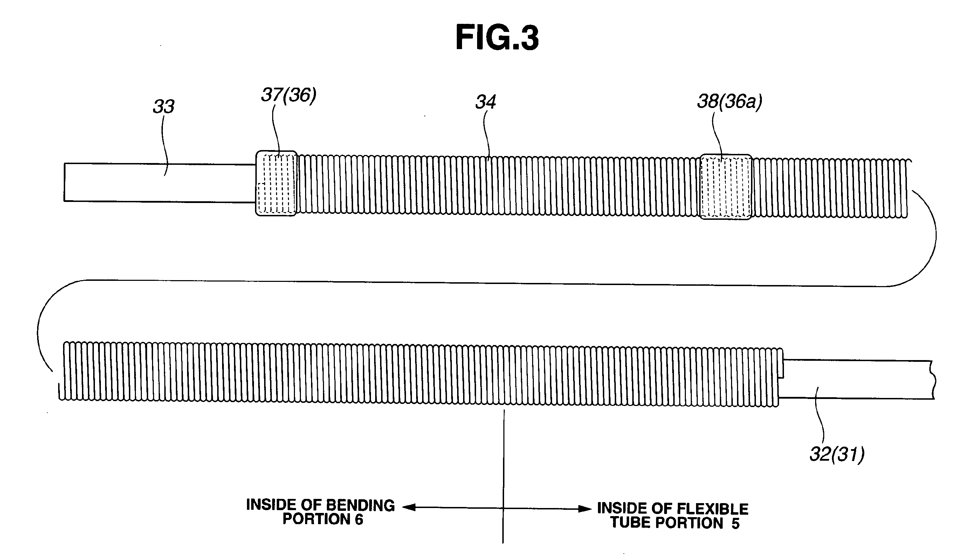

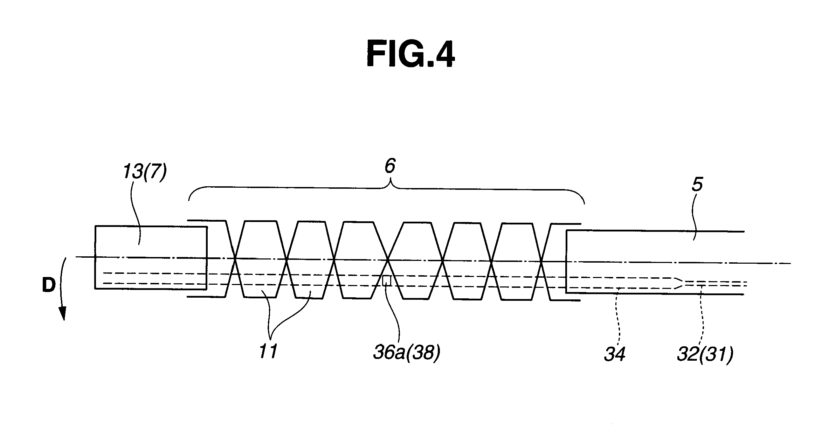

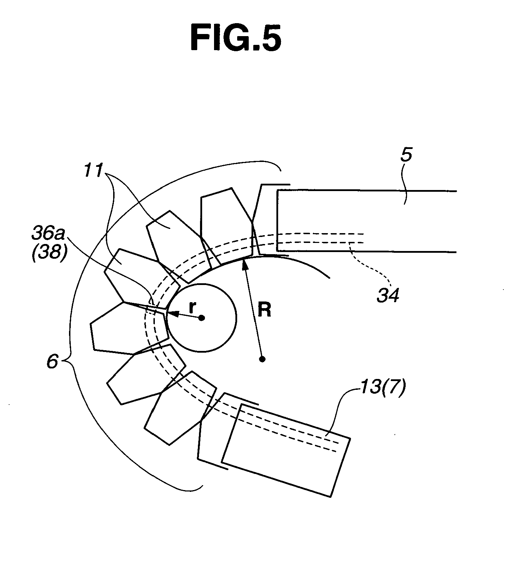

FIGS. 1 to 13 relate to a first embodiment of the present invention, wherein FIG 1 shows an appearance of an endoscope of the first embodiment of the present invention, FIG. 2 shows an internal configuration of a distal end side of an insertion portion, FIG 3 shows a structure to protect a light guide fiber bundle by a protecting coil member, FIG 4 shows that position of an adhesive fixing to one another wires of the protecting coil member shown in FIG 3 is near the center in a longitudinal direction of a bending portion, and FIG 5 shows the bending portion in a maximally bent state is fixed with the adhesive to a position where the radius of curvature of the bending portion is minimized.

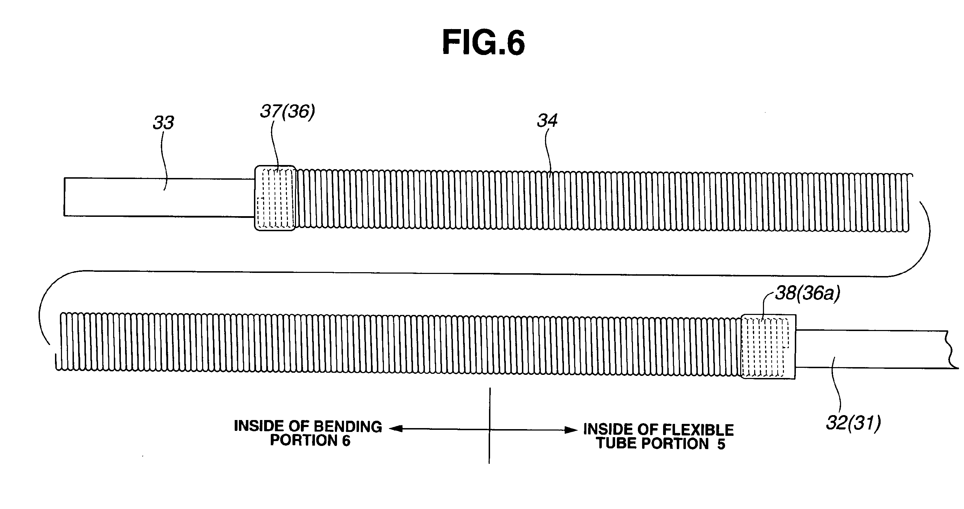

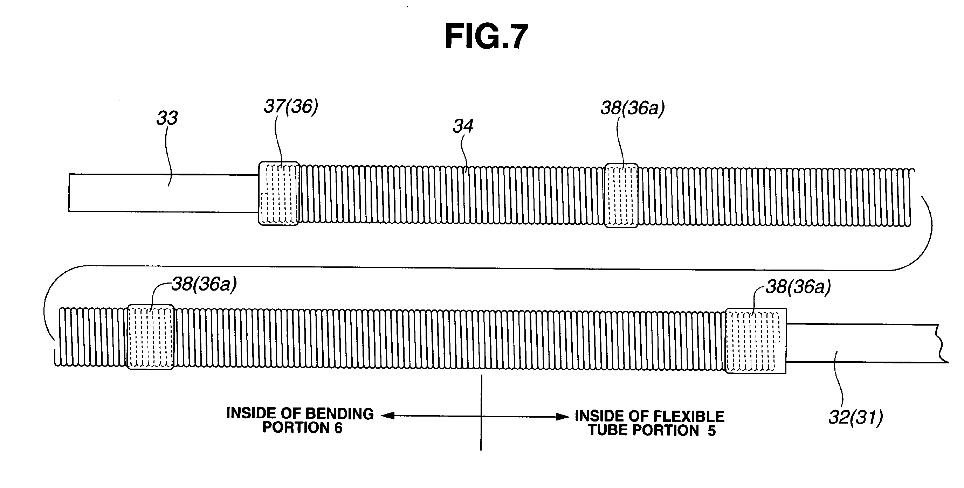

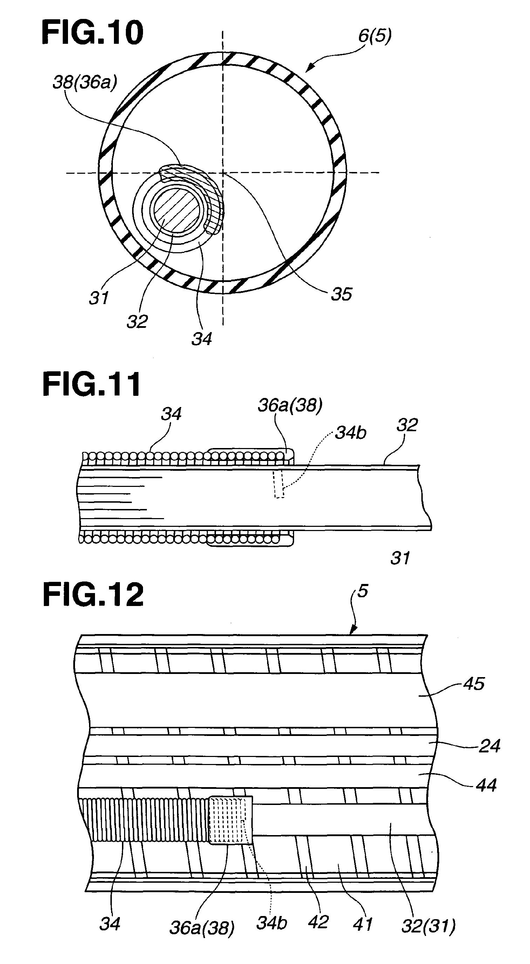

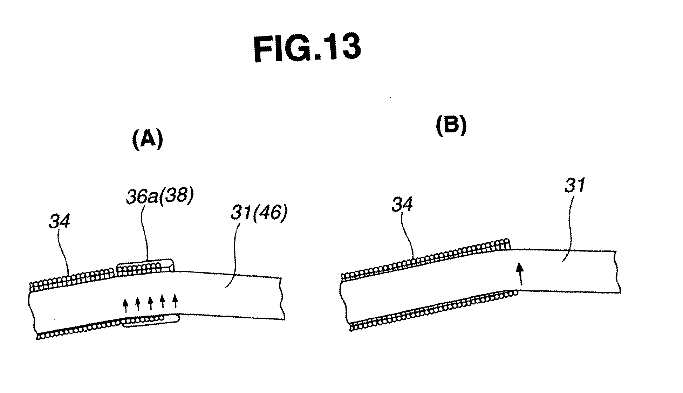

FIGS. 6 and 7 show exemplary fixing positions different from the fixing position with adhesive shown in FIG 3, FIGS. 8 to 10 show structures of a fixing portion in a circumferential direction in fixing the wires of the protecting coil member to one another, FIG 11 shows a structure near a rear end of the protecting coil member, FIG 12 shows the structure near the rear end of the protecting coil member in the flexible tube portion, and FIG 13 shows an illustrative view of workings when a rigid portion is and is not formed.

As shown in FIG 1, an endoscope 1 of the first embodiment of the present invention includes an elongate insertion portion 2 (as an endoscope insertion portion) to be inserted into a body cavity or the like, an operation portion 3 provided to a proximal end of the insertion portion 2, and a universal cable 4 extended from a side portion of the operation portion 3. An end portion of the universal cable 4 is provided with a connector not shown to be detachably connected to a light source apparatus and a signal processing apparatus.

The insertion portion 2 includes a long flexible tube portion 5 which is elongate and flexible, a bending portion 6 which is coupled to a distal end of the flexible tube portion 5 and is bendable or bend-free, and a rigid distal end portion 7 which is coupled to a distal end of the bending portion 6.

The operation portion 3 is provided with bending operation knobs 8. The operator can bend the bending portion 6 in a desired direction by performing an operation to rotate the bending operation knobs 8.

Near a front end of the operation portion 3, a treatment instrument insertion port 9 is provided. The operator can perform a treatment on a diseased part or the like by inserting a treatment instrument from the treatment instrument insertion port 9 through a channel not shown provided in the insertion portion 2 so as to protrude a distal end side of the treatment instrument from a distal end aperture of the distal end portion 7.

FIG 2 shows a structure of the distal end side of the insertion portion 2, that is, structures of a distal end side of the bending portion 6 and the distal end portion 7.

The bending portion 6 has a plurality of (more specifically, many) bending pieces 11 having ring bodies, such that adjacent ones are pivotably coupled by rivets 12. The rivets 12 pivotably couple the adjacent bending pieces 11 at positions corresponding to up/down and left/right directions of the bending portion 6. Note that the bending piece 11 at the distal-most end is secured by a distal end to a distal end portion main body 13 configuring the distal end portion 7.

Inside the plurality of bending pieces 11, 11,..., 11, bending wires 14 are inserted along up/down and left/right positions pivotably coupled by the rivets 12. Distal ends of the bending wires 14 are secured to the bending piece 11 at the distal-most end by brazing or the like. Rear end sides of the bending wires 14 are coupled, in through the insertion portion 2, to a sprocket not shown provided in the inside of the bending operation knob 8 in the operation portion 3.

Further, a bending mechanism is formed such that, by an operator operating to rotate the bending operation knob 8, one of the bending wires 14 in up/down and left/right pairs is towed with the other wire relaxed, so that the bending portion 6 is bent toward the side of the bending wire 14 towed.

Note that, a wire guide 15 is provided on an inner circumferential surface of the bending pieces 11, to regulate insertion position of the bending wire 14 inserted in the bending portion 6.

Outer circumferential surfaces of the plurality of bending pieces 11, 11, ..., 11 are covered with a mesh-tube 16. An outer circumferential surface of the mesh-tube 16 is covered with a covering tube 17 with rich elasticity which is made, for example, of a rubber. A distal end of the covering tube 17 is secured on an outer circumferential surface of the distal end portion main body 13 with a spool and an adhesive. A rear end of the covering tube 17 is secured to a border portion of a distal end of the flexible tube portion 5.

In the insertion portion 2, various contained matters are incorporated as explained below. In the present embodiment, a light guide fiber bundle 31, as a contained matter of a flexible body which is elongate and flexible, is covered with a protecting coil member 34 as a protecting helical body having a helical shape, thus providing contained matter protecting means to adequately protect the light guide fiber bundle 31.

The contained matter protecting means of the present embodiment ensures, by covering the contained matter with the protecting coil member 34, a contained matter protection function for protecting the contained matter covered with the protecting coil member 34, as discussed below.

Further, by adhering and fixing adjacent wires configuring the protecting coil member 34 to one another with adhesive or the like, the rigid portion, which functions as the regulating portion for regulating the flexion amount of the protecting coil member 34, is designed to be settable easily, inexpensively, and at any local position.

In addition, by locally regulating the flexion amount of a part of the light guide fiber bundle 31 which is inside the rigid portion, it is allowed to protect the light guide fiber bundle 31 as the contained matter, while providing buckling prevention or the like to the part for protection. In this manner, the present embodiment allows easily and effectively protecting a part desired of protection by locally regulating the flexion amount if there is such a part.

On a distal end surface of the distal end portion main body 13, there are provided open holes respectively serving as an observation window 18 and an illumination window 19. The open hole forming the observation window 18 is attached with an object optical system 21 via a frame body 20. The frame body 20 is secured to a device frame having an image pickup surface of a solid-state image pickup device 22 arranged at an image forming position of the object optical system 21, thus forming an image pickup unit 23.

On a rear surface side of the solid-state image pickup device 22 is arranged a peripheral circuit portion, such as a driving circuit not shown for driving the solid-state image pickup device 22 and a buffer circuit for amplifying an output signal of the solid-state image pickup device 22, which is connected to a distal end of the signal cable 24 inserted in the insertion portion 2. The solid-state image pickup device 22 and the peripheral circuit portion are covered with an armor member 25. Note that a front surface and an outer circumference on the front surface side of the distal end portion main body 13 are covered with a distal end cover 26 having apertures provided at parts corresponding to the observation window 18 and the illumination window 19.

To the open hole which is open at the illumination window 19 mounted with an illumination lens 27 is secured a distal end side of the light guide fiber bundle 31 as the elongate flexible body which is inserted in the insertion portion 2 and flexed depending on the bending action of the bending portion 6.

A part of the light guide fiber bundle 31 extending from the bending portion 6 to the inside of the flexible tube portion 5 is armored with a flexible armor tube 32 such as, for example, a silicon tube. A distal end part of the light guide fiber bundle 31 is armored by a distal end cap member 33 having a cylindrical shape which is secured to the distal end portion main body 13.

At least in the bending portion 6, an outer circumferential surface of the armor tube 32 covering an outer circumferential surface of the light guide fiber bundle 31 is covered with the protecting coil member 34 as the protecting helical body formed by closely winding, in a constant diameter, a wire having a predetermined elasticity, e.g., a stainless steel wire. The light guide fiber bundle 31 is thus protected by the protecting coil member 34. Note that the protecting coil member 34 is not limited to one formed using a stainless steel wire as the above-mentioned wire, but instead may be a stainless steel strip or the like.

Further, on a distal end side of the protecting coil member 34, a wire end portion 34a is arranged on an outer circumferential side (peripheral side) with respect to a center axis 35 of the bending portion 6, and a part of several windings from the end portion is adhered and fixed on an outer circumferential surface of the distal end cap member 33, with an adhesive 36 or the like. In other words, the distal end of the protecting coil member 34 is fixed to the distal end cap member 33 by a distal end side fixing portion 37 of the adhesive 36.

FIG 3 shows the contained matter protecting means where the protecting coil member 34 is fitted to cover the light guide fiber bundle 31, in the bending portion 6 as the distal end side shown in FIG 2 and in the flexible tube portion 5.

At a part of the protecting coil member 34 where the maximum buckling force is applied to the light guide fiber bundle 31 when the bending portion 6 is bent, there is formed a rigid portion 38 with rigidity that has a function as a regulating portion to locally regulate a flexion amount, in order to restrict the buckling or the like.

Specifically, at least two adjacent threads of wires of the protecting coil member 34 on the outside of the part applied with the maximum buckling force are adhered and fixed to each other with an adhesive 36a or the like on the entire circumference or the like, so as to form the rigid portion 38. Note that the adhesive 36a forming the rigid portion 38 may be one same as the adhesive 36 mentioned above.

FIG 4 is a view to schematically illustrate on which position of the bending portion 6 the adhesive 36a of FIG 3 is placed. In the present embodiment, at least two adjacent threads of wires of the protecting coil member 34 are fixed to each other with the adhesive 36a in the vicinity of the center of the longitudinal direction of the bending portion 6.

FIG 5 is a view of the bending portion 6 of FIG 4 as maximally bent in a downward direction (abbreviated as D direction). In the present embodiment, radius of curvature in the bending portion 6 is not uniform. In the example shown in FIG 5, assuming the radius of curvature at the center position of the longitudinal direction of the bending portion 6 as r, and the radius of curvature on the hand side of the bending portion 6 as R, a relation is obtained as r < R. In the maximally bent state shown in FIG 5, r is the minimum radius of curvature.

Thus, in the present embodiment, the position of the adhesive 36a (rigid portion 38) shown in FIG 4 is approximately identical with the position of the minimum radius of curvature r shown in FIG 5.

In other words, the rigid portion 38 with a certain width on the entire circumference (i.e., the part of the adhesive 36a) is formed on the part of the protecting coil member 34 which covers the part of the light guide fiber bundle 31 which is applied with the maximum buckling force, that is, the part bent at the minimum radius of curvature, when the bending portion 6 is maximally bent in a certain direction. Therefore, the light guide fiber bundle 31 inserted inside of the rigid portion 38 is regulated from bending at a small radius of curvature, allowing effectively preventing buckling occurrence, decrease in life time, and so on.

When unbent and straight as shown in FIG 3, the protecting coil member 34 is wound in a helical shape with adjacent wires in approximately close contact to one another at least in the bending portion 6.

Accordingly, it can be prevented that, at the time of bending, a clearance of a loose winding portion is caught by the bending pieces 11 or the like, to be further enlarged, decreasing the effect of protecting the light guide fiber bundle 31 inserted in the bending pieces 11 as in the case with prior arts.

Note that the present embodiment is not limited to the examples illustrated in FIGS. 3 and 4. The rigid portion 38, formed by binding the wires to each other with the adhesive 36a or the like, may be appropriately set to the minimum, according to the actual situation such as the buckling force applied on the flexible contained matter such as the light guide fiber bundle 31.

The rigid portion 38 is preferably provided at least one location on the hand side except the distal end side fixing portion 37 with the distal end cap member 33.

More specifically, the rigid portion 38 may be provided only in the bending portion 6 as in FIG 3 or only in the flexible tube portion 5 as in FIG 6. The rigid portion 38 may also be provided in both the bending portion 6 and the flexible tube portion 5 as in FIG. 7.

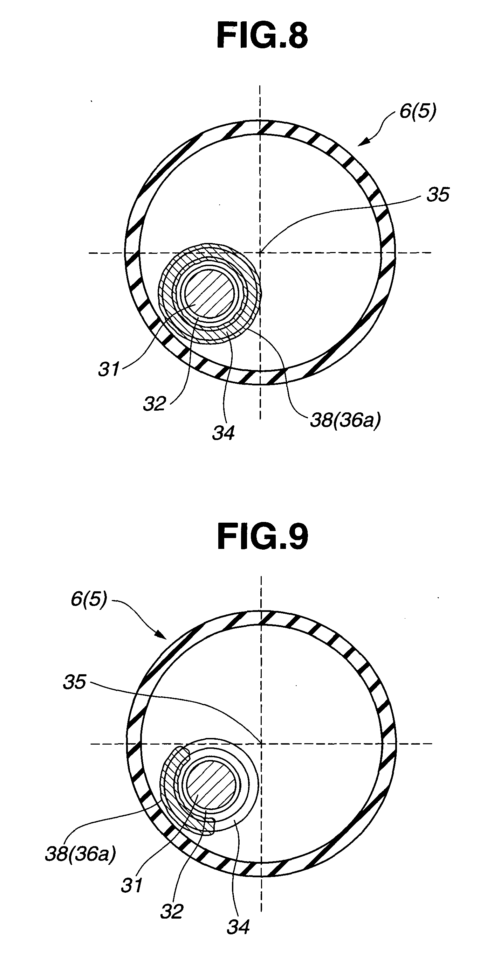

Further, when adhering the adhesive 36a serving as the rigid portion 38 to the wire of the protecting coil member 34, the adhesion range in the circumferential direction is not limited to on the entire circumference as in FIG 8, but may be only on an outer circumferential side with respect to a center axis 35 (in a front/back direction on the paper surface) as in FIG 9, or conversely, on an inner circumferential side with respect to the center axis 35 as in FIG 10. The adhesive 36a may also be adhered at a position and in a range other than those shown in FIGS. 8 to 10.

For example, by forming the rigid portion 38 at a part crossing the radial direction with respect to the center axis 35 where the light guide fiber bundle 31 as the contained matter is arranged as in FIGS. 9 and 10, restriction of the flexion amount in bending in that direction can be realized with a small amount of the rigidly formed part.

It is also made possible to prevent flexion in other directions from being restricted. In other words, according to the present embodiment, it is easily made possible to, with respect to a flexing direction, locally restrict the flexion amount to ensure the contained matter protecting function.

In the case of the endoscope, even with the same flexible contained matter and the same protecting coil member 34, the part where the maximum buckling force is applied to the flexible contained matter minutely differs for each type of the endoscope. By changing the rigid portion 38, which is formed of the adhesive 36a on adjacent wires of the protecting coil member 34 to one other, depending on the actual situation of the each type of the endoscope, the protecting coil member 34 itself can be commonly used, thus realizing at low cost (inexpensively providing) the contained matter protecting means and the endoscope 1 including the contained matter protecting means.

In this case, by applying the adhesive 36a on wires adjacent to one another, it becomes possible to easily form the rigid portion 38 as the regulating portion for regulating the flexion at the part applied with the adhesive 36a, i.e., a local part, which in turn allows realizing at a low cost the contained matter protecting means and the endoscope 1 including the contained matter protecting means.

For example, it can be facilitated to, for the insertion portion of a first type of the endoscope, apply the adhesive 36a on adjacent wires of the protecting coil member 34 to one another on the entire circumference so as to form the rigid portion 38 as shown in FIG 8, while for the insertion portion of a second type of the endoscope, apply the adhesive 36a on adjacent wires of the protecting coil member 34 to one another, partly on the circumference direction so as to form the rigid portion 38 as shown in FIG 9.

Thus, in the present embodiment, the rigid portion 38, which is applied with the adhesive 36a to have the function as the regulating portion for locally regulating the flexion, can be easily changed and set depending on the endoscope type, characteristics of the contained matter, or the like.

For example, as a position and range in which to apply the adhesive 36a, a position and range thereof in the longitudinal direction of the protecting coil member 34 and also a position and angular range thereof in the circumferential direction can be freely set.

In addition, because the protecting coil member 34 in the present embodiment may be closely wound on approximately the entire area at least in the bending portion 6, processing of parts can be facilitated and inexpensive unlike a conventional type of protecting coil member having a loose winding portion and a close winding portion that complicatedly coexist.

Further, the method for fixing the wires adjacent to one another is not limited to using the adhesive 36a, but may use any of solder, laser welding, and so forth. Furthermore, the flexible contained matter fitted and covered with the protecting coil member 34, described as the light guide fiber bundle 31 in the above-described embodiment, may be an elongate and flexible image guide fiber bundle, a fluid pipe tube such as an air/water feeding tube 44 or a suction tube 45 as shown in FIG 12, or other contained matters.

FIG 11 is a cross-sectional view of an enlarged hand side end (rear end) of the protecting coil member 34 shown in FIGS. 6 and 7.

On the protecting coil member 34, the rigid portion 38 is formed by binding several threads of wires including a coil end with the adhesive 36a. Methods to apply the adhesive 36a may include manual application or use of an automatic coating apparatus or the like to form an adhesive layer as in a model molding.

Both the inside and outside of the hand side end of the adhesive 36a are chamfered or rounded.

FIG 12 is a view showing the rear end in FIG 11 present in the flexible tube portion 5.

The innermost surface of the flexible tube portion 5 is normally configured by a helical tube portion 41 formed by winding a stainless steel strip or the like to a helical shape, with a clearance 42 adjacently present in helical shape over approximately the entire length of the helical tube portion 41.

In the flexible tube portion 5 is inserted the flexible armor tube 32 that covers the light guide fiber bundle 31 as the flexible contained matter. The flexible armor tube 32 is further covered by the protecting coil member 34 on the bending portion 6 side, and is secured with a rear end of the protecting coil member 34 with the adhesive 36a in the flexible tube portion 5.

In the flexible tube portion 5 are also inserted, as flexible contained matters, the air/water feeding tube 44, the signal cable 24, the suction tube 45 also used as a channel, and the like.

Despite the clearance 42 formed between adjacent parts of the helical tube portion 41 as described above, because the rear end of the protecting coil member 34 is bound with the adhesive 36a, it can be prevented that a wire end portion 34b is caught by the clearance 42 during assembly, that is, assembly is facilitated.

Even if the protecting coil member 34 is moved forward and backward in an axial direction during a bending operation, the wire end portion 34b is prevented from being caught by the clearance 42, which avoids disturbing the movements of the other flexible contained matters such as the air/water feeding tube 44. In other words, in the present embodiment, the hand side end of the protecting coil member 34 is bound with the adhesive 36a so as not to be exposed, which results in the flexible tube portion 5 to have a good assemblability and a structure to hardly harm other flexible contained matters.

The structure of the present embodiment in which the rear end is bound with the adhesive 36a is also elective for a case shown in FIG 13.

FIG 13 (A) shows a state of the vicinity of the rear end of the protecting coil member 34 of the present embodiment when the flexible tube portion 5 (not shown) is mildly bent along a lumen. FIG 13 (B) shows, for the sake of comparison, a case where the rigid portion is not provided to the rear end of the protecting coil member 34.

As shown in FIG 13 (B), when the rigid portion is not provided to the hand side end portion of the protecting coil member 34, it is possible that the end portion of the protecting coil member 34 damages the light guide fiber bundle 31 by a point as shown in an arrow.

In contrast, in the case of FIG 13 (A), because the end portion with the adhesive 36a of the protecting coil member 34 applies a force by a line or surface, not a point, as shown in an arrow, there is a merit of allowing for a structure that hardly damages the flexible contained matters even for a local bending in the neighborhood of the end portion.

In this case, as seen from the comparison with FIG 13 (B), it is preferable to rigidify at least wires adjacent to one another over a range of not less than 360 degrees (rigidify the part of multiple windings), if the rigid portion 38 is to be formed at the end portion with the adhesive 36a or the like.

Note that the protecting coil member 34, described in FIG 13 (A) to cover the light guide fiber bundle 31, can also be applied to cover the flexible contained matters 46 such as the air/water feeding tube 44. For this reason, in FIG 13(A), a symbol (46) is shown to clearify applicational cases not only to the light guide fiber bundle 31 but to other flexible contained matters 46.

Note that, if the light guide fiber bundle 31 is installed in the distal end portion main body 13, the wire end portion on the hand side of the protecting coil member 34 is arranged on an outer circumferential side with respect to the center axis 35 shown in FIG 2. Therefore, even if the rear end of the protecting coil member 34 is advanced forward and retreated backward in the axial direction by a bending operation, the wire end portion is prevented from badly affecting the other contained matters.

As mentioned above, the present embodiment can be widely applied to such cases as different types or the like of the endoscope or different types of the contained matter, with a simple configuration. It is also enabled to effectively protect the entire part where the contained matter is covered by the protecting coil member 34, and to locally restrict flexion at a part thus effectively preventing occurrence of buckling, etc.

More specifically, even in a configurational case where a part of the protecting coil member 34 is locally bent at a small curvature when the bending portion 6 is bent at the maximum bending angle, it can be easily prevented that the part is flexed more than required and buckled, by securing the wires to one another with the adhesive or the like to form the rigid portion 38 (i.e., the rigid regulating portion). Thus, an endoscope having an inexpensive contained matter protecting means can be provided.

### (Second Embodiment)

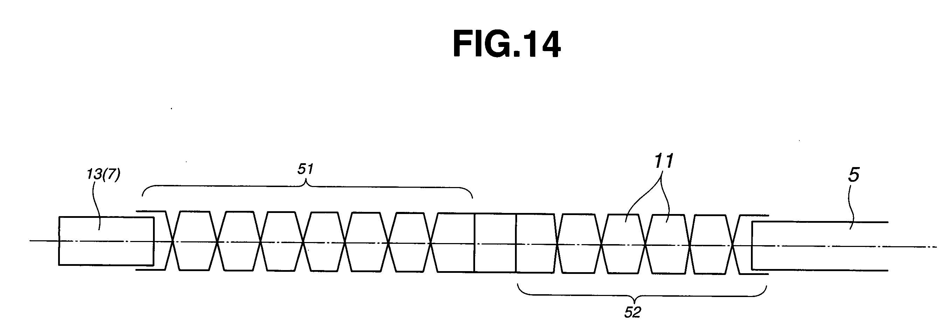

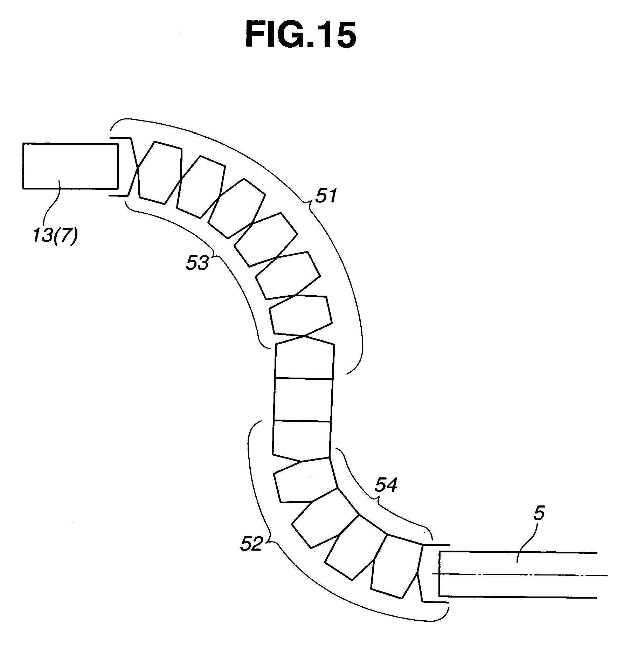

Next, a second embodiment of the present invention is described referring to FIGS. 14 and 15. The present embodiment is basically the same as the first embodiment in configuration except that two bending portions are provided.

FIG 14 schematically shows the bending pieces 11 configuring two parts of a first bending portion 51 and a second bending portion 52 capable of being independently operated for bending.

FIG 15 shows an exemplary state where the first bending portion 51 is bent downward by 90 degrees and the second bending portion 52 upward by 90 degrees. The first bending portion 51 and the second bending portion 52 are designed such that, when both are bent by the same value of 90 degrees, the total of bending clearances 54 on the second bending portion 52 side formed together by the adjacent bending pieces 11 has less allowance than the total of bending clearances 53 on the first bending portion 51 side. FIG 15 shows a part of the bending pieces 11 where the bending clearances 53, 54 are formed.

Though not illustrated in the present embodiment, the hand side end portion of the protecting coil member 34 shown in FIGS. 3, 6, 7 and so on (of the first embodiment) is extended up to the inside of the flexible tube portion 5, as in FIG 4 of the first embodiment. The position of the rigid portion 38 adhered is also provided under a concept similar to that shown in the first embodiment.

The present embodiment has the same effect as in the first embodiment.

An effect unique to the second embodiment is that, because the second bending portion 52 has an allowed bending angle narrower than that of the first bending portion 51, it is restrained that each of the flexible contained matters (not shown) is tended to bend with time thus affecting to cause a problem of an increased angle than an initial angle, and as a result, the damage on the contained matters due to the increased angle with time can be prevented thereby stabilizing quality. Generally, the bending portion side has a smaller radius of curvature than that of the flexible tube portion 5 side. Therefore, by the flexible tube portion 5 side, i.e., the second bending portion 52 having a narrower allowed bending angle, it is better prevented that each of the flexible contained matters is drastically bent at an exit of the flexible tube portion 5. This leads to a greater quality stabilizing effect than is obtained by a structure where the first bending portion 51 has an allowed bending angle narrower than that of the second bending portion 52. Note that an embodiment or the like configured by partial combination or the like of the above-mentioned embodiments also belongs to the present invention.

Thus, in the present invention, at least one of the plurality of flexible contained matters, insertedly arranged from the inside of the bending portion to the inside of the flexible tube portion, is sheathed with the flexible protecting coil member, and the outer surface of the protecting coil member is partially provided with the rigid portion (regulating portion). The rigid portion is provided at the part where a great buckling force is applied on the flexible contained matter when the bending portion or the flexible tube portion is bent, that is, the part where the portion is bent in a small radius of curvature. Accordingly, the flexible contained matter at that part can be protected, regulating application of a great buckling force thereto, thus allowing for a longer life.

### (Third Embodiment)

Next, a third embodiment of the present invention is described. First, background thereof is described. In Japanese Utility Model Laid-Open No. 54-90086, there is disclosed a sliding tube type endoscope insertion assisting tool including a sliding tube as a cylindrically shaped body member that renders insertable an insertion portion or the like of an endoscope, thus assisting insertion thereof into a body cavity or the like, and an inflatable/contractable balloon provided to an outer circumference of a distal end portion of the sliding tube.

The sliding tube type assisting tool needs to have a wide clearance between the endoscope and the sliding tube to improve slidability, resulting in unavoidable increase in the diameter of the sliding tube.

The thickening of the sliding tube results in increase in the radius of curvature of the tube itself, causing not only bending difficulty for a small flexion portion in the body cavity, but also an increased surface area which deteriorates slidability with respect to the intestinal tract, with a final result of insertion of the endoscope into a deep part rendered time-consuming.

In addition, another factor of the lengthened inspection time is the necessity to frequently check, using X-ray or the like, the position, swelling condition, etc. of the balloon because the balloon is always outside the field of view.

Accordingly, the third embodiment describes an endoscope insertion assisting tool and an endoscope apparatus that can achieve an object such as allowing smooth insertion of the insertion portion into the body cavity to shorten inspection time.

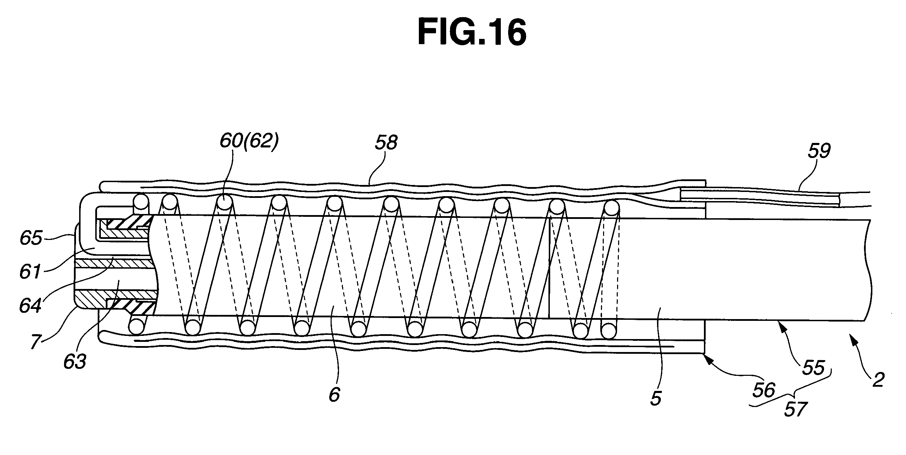

FIG 16 shows an endoscope apparatus 57 including an endoscope of the third embodiment of the present invention. This endoscope apparatus 57 includes an endoscope 55, and an endoscope insertion assisting tool 56 for assisting the insertion of the endoscope 55, which is attached detachably and in a simple manner to the distal end side of the insertion portion 2 of the endoscope 55.

The endoscope insertion assisting tool 56 of the present embodiment is configured mainly of four members: a balloon 58; a fluid supplying tube 59 to supply a fluid such as air to inflate the balloon 58; a balloon holding member 60 to hold the balloon 58; and a shaft 61 to advance forward and retreat backward the balloon 58 and the balloon holding member 60 from the hand side.

The balloon 58 that inflates and contracts by fluid supply and exhaustion is formed in a bag shape with a member having rich expansion and contraction characteristics such as latex, for example. The fluid supplying tube 59 is made of silicon, for example, and connected to an air-feeding pump not shown or a syringe not shown or the like as fluid supplying/exhausting means on a hand side of the tube. Thus, it is enabled to cause the air-feeding pump or the like to supply and suck a fluid such as air into and from the balloon 58 through the fluid supplying tube 59 so as to freely inflate and contract the balloon 58.

The balloon holding member 60 is a flexible member having flexibility, configured of a spring 62 made of teflon (a registered trademark) as a fluorocarbon resin, for example. The spring 62 is set in a size to allow attachment and detachment thereof to outer circumferential surfaces of the distal end portion 7 and the bending portion 6.

The balloon holding member 60, so long as a member having flexibility, is not limited to the spring 62 in terms of material and structure, and may be a substitute member such as, for example, a net-shaped fluorocarbon resin tube, or a fluorocarbon resin tube which is more rigid than the balloon 58 and less inflatable and contractable.

The balloon 58 and the balloon holding member 60 as balloon members detachably attached in the vicinity of the distal end portion 7 of the endoscope 55 are in a holding structure where the balloon 58 is simply expanded and placed on the balloon holding member 60, but may be adhered and fixed with an adhesive.

The shaft 61 as a moving member to move the balloon member is a flexible member, and is a bar member made of fluorocarbon resin, for example. As long as the shaft 61 has flexibility, no limitation is placed on material and structure thereof, and may be a substitute member such as, for example, a coil formed using a stainless steel material.

In the example shown in FIG 16, the shaft 61 is formed in one-piece molding with the same material as that of the balloon holding member 60, but may be a separate body structured to be fixed to the balloon holding member 60 with an adhesive or the like.

In the insertion portion 2 of the endoscope 55, on the other hand, there are formed a first channel 63 for suction and for insertion of the treatment instrument, and a second channel 64 for attachment and advance/retreat operation of the endoscope insertion assisting tool 56. In the second channel 64, a stick-shaped shaft 61 is inserted.

At a distal end side aperture of the second channel 64 is formed a concave portion 65 extending to an outer circumferential surface side. In the state shown in FIG 16, a turn back portion of a shaft 61 configuring the endoscope insertion assisting tool 56 is detachably fitted into the concave portion 65.

Though not illustrated in FIG 16, the distal end of the endoscope 55 is provided, for example, with the observation window, the illumination window, the air/water feeding nozzle, and so forth, as in the first embodiment. As in the first embodiment, the insertion portion 2 of the endoscope 55 includes the bending portion 6 formed at the distal end of the flexible tube portion 5, and the rigid distal end portion 7 provided at the distal end of the bending portion 6. In other words, the endoscope 55 of the third embodiment has a configuration where, basically, the endoscope 1 of the first embodiment is further provided with the second channel 64.

The endoscope apparatus 57 of the present embodiment is configured such that a peripheral portion of the balloon 58 of the endoscope insertion assisting tool 56 is attachably/detachably provided in the vicinity of the distal end portion 7 of the endoscope 55 and the peripheral portion of the balloon 58 is freely movable toward a front side to be caught in a field of view range of the endoscope 55, so as to facilitate an assisting operation of the endoscope insertion, as described below.

The following describes workings of the thus configured endoscope 55 in the present embodiment inserted into a body cavity to perform endoscopy.

Before performing an endoscopy, the endoscope insertion assisting tool 56 is inserted from the distal end side aperture of the second channel 64, and attached to a peripheral portion of the distal end portion 7 of the endoscope 55 as in FIG 16 (near outer circumferences of the distal end portion 7 and the bending portion 6 in FIG 16).

Note that, though not shown, a rear end of the shaft 61 of the endoscope insertion assisting tool 56 is introduced to the outside at the operation portion (not shown) of the endoscope 55, such that the operator can grasp and advance/retreat the shaft 61.

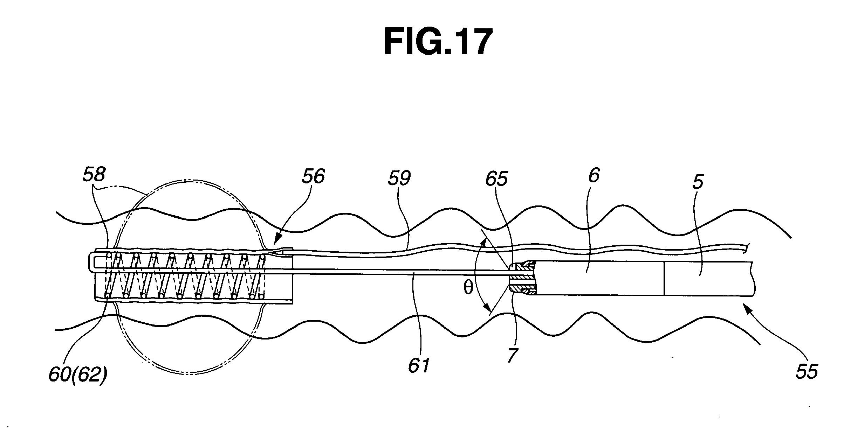

When inspecting the inside of the body cavity, the balloon 58 in a contracted state as shown in FIG 16 is inserted into the body cavity from the distal end side of the endoscope 55. The distal end of the endoscope 55, however, may thereafter have difficulty in advancing in the body cavity. The following describes an operation method of the present embodiment for such case referring to FIG 17 to 21.

First, the operator pushes out the shaft 61 to move the balloon 58 from over the bending portion 6 to the front side of the field of view as shown in FIG 17. A symbol θ shown in FIG 17 represents a field of view range of the observation window.

Next, air 66 is supplied into the balloon 58 through the fluid supplying tube 59, so that the balloon 58 is inflated to be held and fixed inside an intestinal tract as shown in a double dotted line.

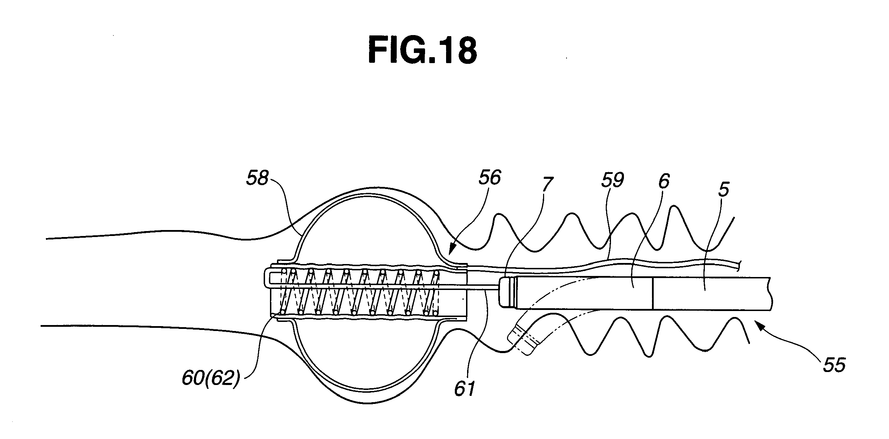

Then, as shown in FIG 18, the operator operates to slowly draw the shaft 61 to haul the intestinal tract by means of the balloon 58 inflated with the air 66.

Next, the operator lightly bends the bending portion 6 of the endoscope 55 as shown in a double dotted line to cause the bending portion 6 to hold the intestinal tract hauled by the balloon 58, thus prohibiting the same from being returning to the original state.

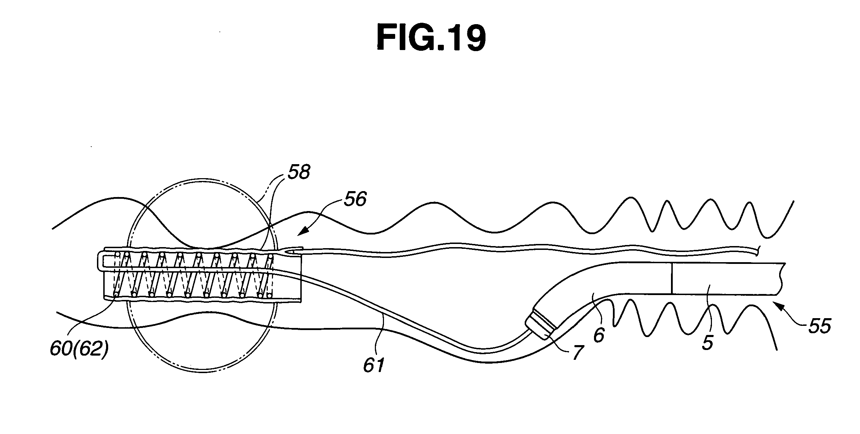

In the holding state, the operator contracts the balloon 58, and again operates to push the balloon 58 forward into the field of view. FIG 19 shows the state where the balloon 58 is thus shrunk (contracted) and again pushed forward into the field of view.

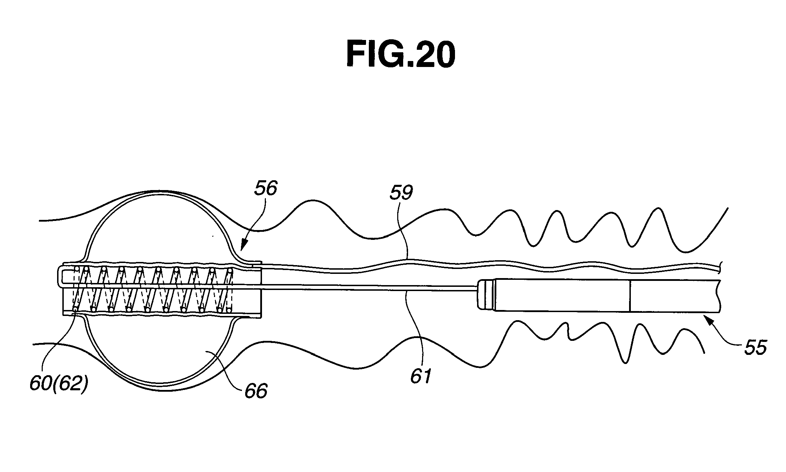

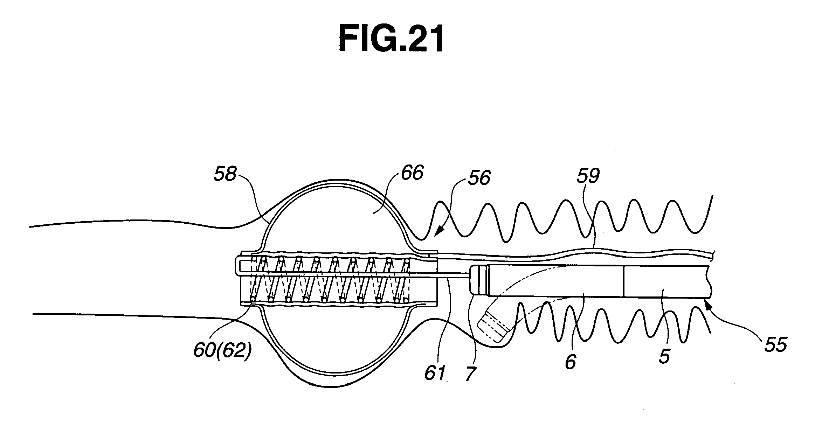

From the state of FIG 19, the operator further inflates the balloon 58 and unbends the bending portion 6 into a state where the intestinal tract is about to be hauled again. This state is shown in FIG 20. With the balloon 58 inflated with the air 66 as shown in FIG 20, the operator operates to slowly draw the shaft 61 so as to haul the intestinal tract by means of the inflated balloon 58. The operator then hauls the intestinal tract by means of the inflated balloon 58 as shown in FIG 21.

By repeating such operations, the operator can advance the distal end position of the endoscope 55 to a relatively deep part of the intestinal tract in a short period of time.

Through occasional operations to slowly draw both the endoscope 55 and the shaft 61 of the endoscope insertion assisting tool 56 together toward the hand side, with the balloon 58 held to the intestinal tract, the operator can eliminate deflection and loop of the intestinal tract to better linearize the intestinal tract.

The active linearization not only facilitates the operator to conduct a force to the distal end of the endoscope 55, but also causes the shaft 61 to function also as an insertion guide of the endoscope 55, allowing it to advance the distal end of the endoscope 55 to the deep part of the intestinal tract not only by hauling the intestinal tract but also by the progression of the endoscope 55 (illustration thereof is omitted).

By repeating a several times combination of the hauling and linearization of the intestinal tract, the operator finally inserts the endoscope 55 to the deepest portion of the intestinal tract.

According to the present embodiment, the shaft 61 of the endoscope insertion assisting tool 56 can be made significantly thinner compared to a shaft of the sliding tube type, which results in a small frictional force with the endoscope 55, thus enabling the advance/retreat operation to be performed with a light level of force amount to insert the treatment instrument.

Moreover, because the radius of curvature of the shaft 61 itself is smaller compared to that of the sliding tube, the endoscope 55 can be easily passed through the flexion portion having a small radius of curvature in the body cavity.

In addition, when inflating the balloon 58 to fix the same in the intestinal tract, the operator can always check the balloon 58 in the field of view and therefore can perform inspection more easily and in a shorter time period.

Further, the operator can move the balloon 58 and the balloon holding member 60 to the outside of the field of view by completely drawing the shaft 61 or by completely pushing the endoscope 55 with the shaft 61 serving as a guide. The resultant wide field of view permits the operator to efficiently perform an observation or treatment.

Furthermore, because the balloon holding member 60 and the shaft 61 are configured of the flexible members as mentioned above, the operator can perform the bending operation of the endoscope 55 without any hindrance even if the balloon 58 is accommodated over the bending portion 6.

Still further, the accommodability of the balloon 58 over the bending portion 6 allows use of a large size balloon having a greater fixing power to the intestinal tract compared to a balloon such as a balloon catheter which is used inserted into a treatment instrument insertion channel from the hand side. The operator can mount the large sized balloon 58 having the greater fixing power over the outer circumferential surface of the bending portion 6. The mounting of the balloon 58 can be performed in a simple and easy manner only requiring the operator to insert the shaft 61 from the distal end side.

Yet still further, even in a state where the balloon 58 is pushed forward to be sufficiently placed in the field of view, the operator can perform the bending operation of the endoscope 55, which facilitates it to delicately control the position of the balloon 58 through the bending operation and insert the balloon 58 into the deeper part.

Because the endoscope 55 is not of the sliding tube type, push/pull and twisting operations of the endoscope 55 are not disturbed by the sliding tube, which leads to ease of operation for the operator.

In the attaching state shown in FIG 16, a part of the endoscope insertion assisting tool 56 is fitted into the concave portion 65 provided to the distal end portion 7 of the endoscope 55, thereby positioning the endoscope insertion assisting tool 56 in a revolving direction (circumferential direction). Therefore, according to the present embodiment, even in twisting operation by the operator in inserting the endoscope 55, the endoscope insertion assisting tool 56 does not rattle and can be inserted integrally with the endoscope 55.

Note that the shaft 61 may be connected with an apparatus that revolves the shaft 61 with the balloon 58 shrunk or completely inflated. Revolving the balloon 58 decreases the frictional resistance with the intestinal tract, thus allowing pushing the balloon 58 into the deeper part than when the balloon 58 is not revolved.

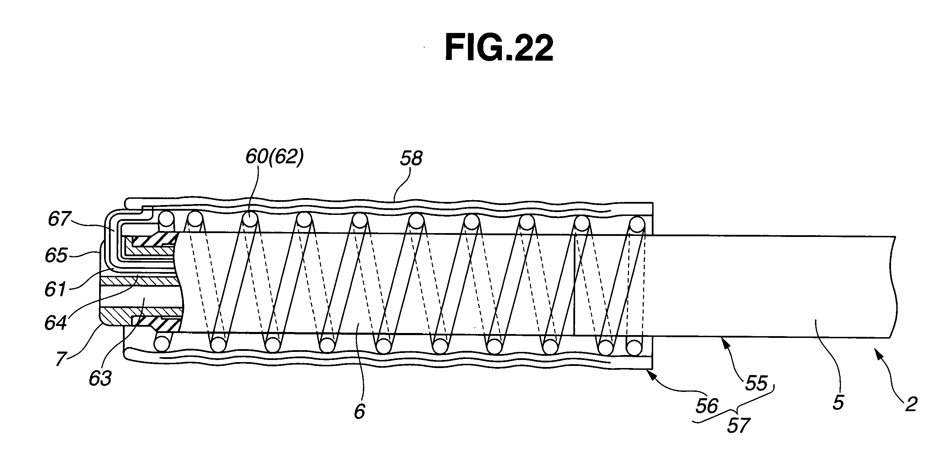

FIG 22 shows the endoscope 55 in an example modified from that of FIG 16. In this modification example, the fluid supplying tube 59 shown in FIG 16 is not provided, but instead, a fluid supplying pipe 67 is formed in the shaft 61.

The other configurations are the same as those shown in FIG 16. The working of this modification example is approximately the same as in the third embodiment shown in FIG 16.

Effects of this modification example includes, in addition to all those described in the third embodiment, that the endoscope 55 can be more easily inserted than in the third embodiment because the fluid supplying tube 59 is not present outside the endoscope 55.

Note that, as a modification example of FIG 16, the shaft 61 as a moving member may be inserted into the fluid supplying tube 59.

### Industrial Applicability

The flexible contained matter such as the light guide fiber bundle having flexibility to be inserted into the insertion portion provided with the bending portion is covered by the protecting helical body such as the protecting coil member. Furthermore, for a part of the flexible contained matters that is easily subject to local buckling or the like, there is provided the regulating portion for regulating the flexion which is obtained by, for example, adhering and fixing to one another with the adhesive, adjacent wires configuring the protecting helical body covering that part. Thus, an endoscope is realized that is usable for a long period of time in spite of the bending of the bending portion.

This application is filed claiming priority from Japanese Patent Application No. 2005-223192 applied on August 1, 2005, the disclosed contents of which being incorporated in the present specification, claims, and drawings.

## Claims

1. An endoscope, comprising:
an elongate insertion portion including a bendable bending portion;
an elongate flexible body as a contained matter disposed in the insertion portion, which is flexed according to a bending action of the bending portion;
a protecting helical body in a helical shape formed by closely winding a wire having a predetermined elasticity, for sheathing and protecting at least a part of the flexible body that is flexed; and
a regulating portion for regulating a flexion of the protecting helical body, formed by securing adjacent wires of the protecting helical body to one another.

2. The endoscope according to claim 1, wherein the regulating portion is provided at a position in an inside of the bending portion, the position corresponding to a position having the minimum radius of curvature when the bending portion is bent.

3. The endoscope according to claim 1, wherein the bending portion is formed by a plurality of ring bodies pivotably coupled to one another, and the regulating portion is arranged in an inside of the ring body, the inside corresponding to a part having the minimum radius of curvature when the bending portion is bent.

4. The endoscope according to claim 1, comprising a rigid portion formed by securing to one another adjacent wires at a rear end portion of the protecting helical body over a range of not less than 360 degrees.

5. The endoscope according to claim 1, wherein the regulating portion is a local rigid portion formed by securing to one another a plurality of adjacent wires in a longitudinal direction of the protecting helical body.

6. The endoscope according to claim 1, wherein the regulating portion is a local rigid portion formed by securing to one another adjacent wires of the protecting helical body over a predetermined angular range in a circumferential direction of the protecting helical body.

7. The endoscope according to claim 1, wherein the regulating portion is formed by a wire part where the adjacent wires are secured to one another by application of an adhesive.

8. The endoscope according to claim 1, wherein the regulating portion is formed by a wire part where the adjacent wires are secured to one another by soldering or laser welding.

9. The endoscope according to claim 1, wherein the regulating portion can be arbitrarily set in terms of a position in the longitudinal direction and a range of the position, or a position in a circumferential direction and a range of the position, of the protecting helical body.

10. The endoscope according to claim 1, wherein the flexible body includes at least one of a fiber bundle and a tube.

11. An endoscope insertion portion, comprising:
an elongate insertion portion including
a distal end portion provided with an illumination window and an observation window,
a bending portion that is bendable provided at a rear end of the distal end portion, and
a flexible tube portion which is elongate and has flexibility, provided at a rear end of the bending portion in a longitudinal direction of the bending portion;
an elongate flexible body as a contained matter disposed in the insertion portion, which is flexed according to a bending action of the bending portion;
a protecting helical body in a helical shape formed by closely winding a wire having a predetermined elasticity, for sheathing and protecting at least a part of the flexible body that is flexed; and
a regulating portion for regulating a flexion of the protecting helical body, formed by securing adjacent wires of the protecting helical body to one another.

12. The endoscope insertion portion according to claim 11, wherein the regulating portion is provided at a position in an inside of the bending portion, the position corresponding to a position having the minimum radius of curvature when the bending portion is bent.

13. The endoscope insertion portion according to claim 11, wherein the bending portion is formed by a plurality of ring bodies pivotably coupled to one another, and the regulating portion is arranged in an inside of the ring body, the inside corresponding a part having the minimum radius of curvature when the bending portion is bent.

14. The endoscope insertion portion according to claim 11, comprising a rigid portion formed by securing to one another adjacent wires at a rear end portion of the protecting helical body over a range of not less than 360 degrees.

15. The endoscope insertion portion according to claim 11, wherein the regulating portion is a local rigid portion formed by securing to one another a plurality of adjacent wires in a longitudinal direction of the protecting helical body.

16. The endoscope insertion portion according to claim 11, wherein the regulating portion is a local rigid portion formed by securing to one another adjacent wires of the protecting helical body over a predetermined angular range in a circumferential direction of the protecting helical body.

17. The endoscope insertion portion according to claim 11, wherein the regulating portion is formed by a wire part where the adjacent wires are secured to one another by application of an adhesive.

18. The endoscope insertion portion according to claim 11, wherein the regulating portion is formed by a wire part where the adjacent wires are secured to one another by soldering or laser welding.

19. The endoscope insertion portion according to claim 11, wherein the regulating portion can be arbitrarily set in terms of a position in the longitudinal direction and a range of the position, or a position in a circumferential direction and a range of the position, of the protecting helical body.

20. The endoscope insertion portion according to claim 11, wherein the flexible body includes at least one of a fiber bundle and a tube.
